# EUROPEAN PATENT APPLICATION

(11) **EP 4 134 400 A1**
(43) Date of publication of application: **15.02.2023**
(21) Application number: 21785484.3
(22) Date of filing: 05.04.2021
(51) Int. Cl.: C08L 33/02, C08F 20/06, C09K 3/00

(54) **VISCOUS COMPOSITION**

(30) Priority: 09.04.2020 JP 2020070572
(71) Applicant: Sumitomo Seika Chemicals Co., Ltd., Kako-gun, Hyogo 675-0145 (JP)
(72) Inventor: YAMAGUCHI, Hirofumi, Himeji-shi, Hyogo 672-8076 (JP); SAKAI, Yuka, Himeji-shi, Hyogo 672-8076 (JP)
(74) Representative: Vossius & Partner Patentanwälte Rechtsanwälte mbB
(86) International application number: PCT/JP2021/014462
(87) International publication number: WO 2021/206042

(57) **Abstract**

Provided is a composition containing a polymer obtained by polymerizing a water-soluble unsaturated carboxylic acid monomer in the presence of a water-soluble sucrose allyl ether, with a low pH and a high viscosity. More specifically, provided is a composition containing (A) a polymer obtained by polymerizing a water-soluble unsaturated carboxylic acid monomer in the presence of a water-soluble sucrose allyl ether, (B) a polymer obtained by polymerizing at least acrylic acid and/or methacrylic acid in the presence of pentaerythritol allyl ether, wherein the amount of the pentaerythritol allyl ether is 0.1 parts by mass or more per 100 parts by mass of the acrylic acid and/or methacrylic acid, and water, the composition having a pH of 5.2 or lower and a viscosity of 1000 mPa·s or higher.

## Description

### Technical Field

The present disclosure relates to a viscous composition.

### Background Art

A polymer obtained by polymerizing a water-soluble unsaturated carboxylic acid monomer in the presence of a water-soluble sucrose allyl ether is known to be useful, for example, as a thickening agent for cosmetic materials (e.g., PTL 1).

### Citation List

### Patent Literature

PTL 1: JP2015-151336A

### Summary of Invention

### Technical Problem

The present inventors found that a composition containing a polymer obtained by polymerizing a water-soluble unsaturated carboxylic acid monomer in the presence of a water-soluble sucrose allyl ether readily loses its viscosity when adjusted to a low pH (e.g., a pH of 5.2 or lower) with the addition of a pH adjuster commonly used in the field of cosmetics (e.g., low-molecular organic acid compounds). As noted above, the composition is useful as a viscous cosmetic composition. However, the decrease in viscosity with a decrease in pH makes it difficult to use the composition in cosmetics that require a low pH (e.g., astringent cosmetic materials and exfoliating gels) while maintaining the desired viscosity.

Thus, the inventors conducted further research to find a technique of decreasing the pH while limiting the decrease in viscosity of the composition.

### Solution to Problem

The inventors found that it could be possible to decrease the pH of the polymer-containing composition while limiting the decrease in the viscosity of the composition when adjusting the pH of the composition (in particular, adjusting it to a low pH), by using a specific amount of an acrylic acid polymer crosslinked by a specific crosslinking agent, instead of a pH adjuster commonly used in the field of cosmetics (e.g., low-molecular organic acid compounds). The inventors then conducted further research.

The present disclosure includes, for example, the subject matter described in the following items.
Item 1. A composition comprising
   (A) a polymer obtained by polymerizing a water-soluble unsaturated carboxylic acid monomer in the presence of a water-soluble sucrose allyl ether,
   (B) a polymer obtained by polymerizing at least acrylic acid and/or methacrylic acid in the presence of pentaerythritol allyl ether, wherein the amount of the pentaerythritol allyl ether is 0.1 parts by mass or more per 100 parts by mass of the acrylic acid and/or methacrylic acid, and water,
   the composition having a pH of 5.2 or lower and a viscosity of 1000 mPa·s or higher.
Item 2. The composition according to item 1, wherein the water-soluble unsaturated carboxylic acid monomer in component (A) is at least one member selected from the group consisting of acrylic acid, methacrylic acid, maleic acid, maleic anhydride, fumaric acid, crotonic acid, and itaconic acid.
Item 3. The composition according to item 1 or 2, wherein the amount of the pentaerythritol allyl ether in component (B) is 0.1 to 1 parts by mass per 100 parts by mass of the acrylic acid and/or methacrylic acid.
Item 4. The composition according to any one of items 1 to 3, having a pH of 4 to 5.2.
Item 5. The composition according to any one of items 1 to 4, having a viscosity of 1000 to 50000 mPa·s.
Item 6. The composition according to any one of items 1 to 5, wherein the amount of the water-soluble sucrose allyl ether in component (A) is 0.01 to 1 parts by mass per 100 parts by mass of the water-soluble unsaturated carboxylic acid monomer.
Item 7. The composition according to any one of items 1 to 6, wherein component (B) is the following copolymer (B-1) and/or copolymer (B-2):
   (B-1) a copolymer obtained by polymerizing the following (i) and (ii), and
   (B-2) a copolymer obtained by polymerizing the following (i), (ii), and (iii);
      (i) 100 parts by mass of (meth)acrylic acid,
      (ii) 0.1 to 1 parts by mass of pentaerythritol allyl ether, and
      (iii) 0.5 to 5 parts by mass of (meth)acrylic acid alkyl ester wherein the alkyl has 10 to 30 carbon atoms.
Item 8. A pH adjuster for a composition,
   the pH adjuster comprising (B) a polymer obtained by polymerizing a water-soluble unsaturated carboxylic acid monomer in the presence of pentaerythritol allyl ether, wherein the amount of the pentaerythritol allyl ether is 0.1 parts by mass or more per 100 parts by mass of the water-soluble unsaturated carboxylic acid monomer,
   the composition comprising (A) a polymer obtained by polymerizing a water-soluble unsaturated carboxylic acid monomer in the presence of a water-soluble sucrose allyl ether.

### Advantageous Effects of Invention

A composition is provided that contains a polymer obtained by polymerizing a water-soluble unsaturated carboxylic acid monomer in the presence of a water-soluble sucrose allyl ether, and that has a low pH and a high viscosity.

### Brief Description of Drawings

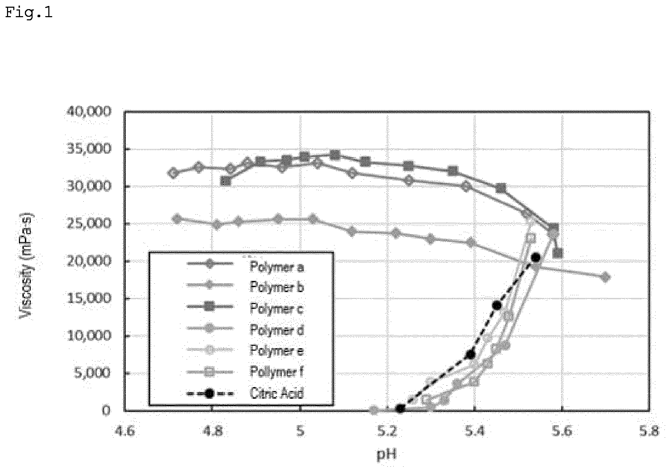

Fig. 1 shows the measurement results of viscosity of polymers when the pH of each polymer obtained by polymerizing a water-soluble unsaturated carboxylic acid monomer in the presence of a water-soluble sucrose allyl ether was adjusted with different compounds.

### Description of Embodiments

The following describes embodiments included in the present disclosure in more detail. The present disclosure preferably encompasses, but is not limited to, a composition that contains a polymer obtained by polymerizing a water-soluble unsaturated carboxylic acid monomer in the presence of a water-soluble sucrose allyl ether and that has a low pH and a high viscosity, the use of the composition, and the method for producing the composition. The present disclosure encompasses all that is disclosed in the present specification and would be recognized by a person skilled in the art.

The composition encompassed by the present disclosure contains (A) a polymer obtained by polymerizing a water-soluble unsaturated carboxylic acid monomer in the presence of a water-soluble sucrose allyl ether, (B) a polymer obtained by polymerizing at least acrylic acid and/or methacrylic acid in the presence of pentaerythritol allyl ether, wherein the amount of the pentaerythritol allyl ether is 0.1 parts by mass or more per 100 parts by mass of the acrylic acid and/or methacrylic acid, and water. The composition has a pH of 5.2 or lower and a viscosity of 1000 mPa·s or higher. The composition encompassed by the present disclosure may also be referred to as "the composition of the present disclosure."

Polymer (A) is, as described above, a polymer obtained by polymerizing a water-soluble unsaturated carboxylic acid monomer in the presence of a water-soluble sucrose allyl ether. The polymer can be obtained, for example, by a method including the step of polymerizing a water-soluble unsaturated carboxylic acid monomer in the presence of a water-soluble sucrose allyl ether according to suspension polymerization. Of the techniques of suspension polymerization, reversed-phase suspension polymerization is preferable: in reversed-phase suspension polymerization, a polymerization reaction is performed by dispersing droplets of an aqueous phase containing a water-soluble unsaturated carboxylic acid monomer, water-soluble sucrose allyl ether, and water in a hydrophobic solvent. In this polymerization, the water-soluble sucrose allyl ether can act as a water-soluble crosslinking agent.

Examples of the water-soluble unsaturated carboxylic acid monomer include, but are not limited to, acrylic acid, methacrylic acid, maleic acid, maleic anhydride, fumaric acid, crotonic acid, and itaconic acid. Of these, acrylic acid and methacrylic acid are preferable. The water-soluble unsaturated carboxylic acid monomers can be used singly, or in a combination of two or more.

The degree of etherification of the water-soluble sucrose allyl ether is preferably 1.8 to 3.5, and more preferably 2.0 to 3.2. The degree of etherification refers to the average molar ratio of allyl ether groups to sucrose. The degree of etherification can be calculated from the amount of acetic anhydride consumed when the hydroxy groups remaining in sucrose allyl ether are reacted with acetic anhydride in pyridine.

The water-soluble sucrose allyl ether can be obtained, for example, by adding sodium hydroxide (catalyst) to an aqueous sucrose solution to convert the sucrose into alkaline sucrose, and performing etherification by adding allyl bromide dropwise. The water-soluble sucrose allyl ether can be obtained efficiently by adjusting the amount of allyl bromide to 2- to 6-fold mol, and preferably 2- to 5-fold mol based on sucrose. The reaction temperature for etherification is, for example, about 80°C. The reaction is typically complete in about 3 hours after the dropwise addition of allyl bromide. The water-soluble sucrose allyl ether can be recovered by adding alcohol to the aqueous phase separated from the reaction solution, filtering off precipitated salts, and distilling off excess alcohol and water.

The hydrophobic solvent for use in reversed-phase suspension polymerization can be, for example, a petroleum-based hydrocarbon solvent selected from the following: aliphatic hydrocarbons, alicyclic hydrocarbons, and aromatic hydrocarbons. Aliphatic hydrocarbons include n-pentane, n-hexane, and n-heptane. Alicyclic hydrocarbons include cyclopentane, methylcyclopentane, cyclohexane, and methylcyclohexane. Aromatic hydrocarbons include benzene, toluene, and xylene. In particular, at least one hydrophobic solvent selected from n-hexane, n-heptane, cyclohexane, and toluene is suitably used as an industrial, versatile solvent. The proportion of the hydrophobic solvent is, for example 100 to 200 parts by mass, per 100 parts by mass of the aqueous phase containing the water-soluble unsaturated carboxylic acid monomer and other components.

In reversed-phase suspension polymerization, the aqueous phase containing the water-soluble unsaturated carboxylic acid monomer and other components, or the hydrophobic solvent, may contain additional components such as a surfactant and a radical initiator.

The surfactant is used primarily for stabilizing the state of suspension during the polymerization. The surfactant can be any surfactant commonly used in reversed-phase suspension polymerization. The surfactant for use is preferably one surfactant, or two or more surfactants, selected from the following: sorbitan fatty acid esters, polyglycerol fatty acid esters, sucrose fatty acid esters, sorbitol fatty acid esters, modified polyethylene wax, modified polypropylene wax, polyvinyl alcohol, polyethylene oxide, cellulose ethers (e.g., hydroxyethyl cellulose and ethylcellulose), sodium alkylbenzene sulfonate, and polyoxyethylene alkylphenyl ether sulfate.

The amount of the surfactant is preferably 0.1 to 10 mass%, and more preferably 0.5 to 5 mass% of the water-soluble unsaturated carboxylic acid monomer. A small amount of a surfactant may possibly cause suspension stability problems during the polymerization. A large amount of a surfactant may be economically disadvantageous.

The radical initiator for use can be any radical initiator commonly used in radical polymerization, and can be preferably, for example, potassium persulfate, ammonium persulfate, sodium persulfate, and an azo initiator. For example, 2,2'-azobis(2-methylpropionamidine) dihydrochloride can be used as a radical initiator.

The amount of the radical initiator is preferably 0.01 to 0.5 mass%, and more preferably 0.02 to 0.2 mass% of the water-soluble unsaturated carboxylic acid monomer. An amount of the radical initiator within these ranges allows the polymerization reaction to more efficiently proceed, and provides better thickening properties when the obtained polymer is used as a hydrophilic thickening agent.

In reversed-phase suspension polymerization, the size of the droplet containing the water-soluble unsaturated carboxylic acid the monomer and other components is closely related to the size of the resulting polymer particles. Although it depends on conditions such as the reactor and production scale, if a 2-L flask, for example, is used as a reactor, polymer particles of appropriate size are likely to be obtained by performing reversed-phase suspension polymerization at a stirring rate of 800 to 1000 rpm. The median particle size of the resulting resin particles can be adjusted by adjusting the stirring rate during polymerization reaction to control the size of the polymer particles (resin particles). For example, resin particles of 5 to 30 um can be obtained. The shape of the polymer particles thus obtained is spherical and maintained in aqueous solutions or viscous materials such as cosmetics. This is considered to have a positive effect on various properties, tactile sensations, and usability in cosmetics containing these particles.

The median particle size of the particles refers to the median particle size of volume average particle size as determined by dispersing the particles in n-hexane and performing measurement by laser diffractometry. For example, a SALD-2000A analyzer (Shimadzu Corporation) can be used as the measurement device for this method (laser diffraction particle size distribution analyzer).

The particles have a median particle size of preferably 5 to 30 pm, more preferably 5 to 25 pm, and still more preferably 6 to 20 pm.

Other conditions of the polymerization reaction, such as the amount of the radical initiator, the temperature of polymerization reaction, and the reaction time, are also adjusted accordingly. The temperature of polymerization reaction is, for example, 50 to 80°C, and the reaction time is, for example, 30 minutes to 3 hours. For example, if a 2-L flask is used as a reactor, the bath temperature can be adjusted to 60°C to initiate a polymerization reaction. In this case, the start of the polymerization reaction can be confirmed with the temperature in the reactor rising to about 70°C with the heat of polymerization. A subsequent aging reaction of 30 minutes to 3 hours typically completes the polymerization reaction. If the aging time is shorter than that, the reaction may not be sufficiently complete, resulting in a large amount of the water-soluble unsaturated carboxylic acid monomer remaining. After the aging reaction, the product can be collected by increasing the bath temperature to distill off the water and petroleum-based hydrocarbon solvent in the reactor.

The degree of neutralization of the polymer can be easily adjusted by neutralizing the carboxyl groups of the water-soluble unsaturated carboxylic acid with an alkali. The phrase "degree of neutralization" as used here refers to the percentage of the number of moles of neutralized groups of the total number of moles of carboxyl groups of the water-soluble unsaturated carboxylic acid. Examples of alkalis for use in neutralization include sodium hydroxide, potassium hydroxide, triethanolamine, and diisopropylamine. The neutralization method is not particularly limited and is, for example, a method of neutralizing the water-soluble unsaturated carboxylic acid monomer beforehand, or a method of neutralizing the polymer obtained by polymerization.

The degree of neutralization of polymer (A) is, for example, but not particularly limited to, preferably 70% or less or 60% or less, more preferably 50% or less or 45% or less, and still more preferably 40% or less.

The amount of the water-soluble sucrose allyl ether is, for example, but not particularly limited to, preferably 0.01 to 1 parts by mass, more preferably 0.05 to 0.8 parts by mass, and still more preferably 0.1 to 0.6 parts by mass, per 100 parts by mass of the water-soluble unsaturated carboxylic acid monomer.

Although not particularly limited, the median particle size of the particles of polymer (A) is preferably about 8 to 10 times larger than that before the particles are blended because the particles absorb water and swell in the composition of the present disclosure. The median particle size of the particles that have absorbed water is preferably about 40 to 300 um, and more preferably about 50 to 250 µm. The median particle size refers to the median particle size of volume average particle size as measured by laser diffractometry. For example, a SALD-2000A analyzer (Shimadzu Corporation) can be used as the measurement device (laser diffraction particle size distribution analyzer) for this method.

Polymer (B) is, as described above, a polymer obtained by polymerizing at least acrylic acid and/or methacrylic acid in the presence of pentaerythritol allyl ether. The phrase "acrylic acid and/or methacrylic acid" indicates that either acrylic acid or methacrylic acid may be used alone, or these can be used in combination; the phrase is synonymous with "(meth)acrylic acid."

Polymer (B) can be obtained, for example, by a method including polymerizing at least (meth)acrylic acid in the presence of pentaerythritol allyl ether. The amount of the pentaerythritol allyl ether for use is 0.1 parts by mass or more per 100 parts by mass of (meth)acrylic acid, as described above. An amount of the pentaerythritol allyl ether considerably smaller than 0.1 parts by mass is not preferable because the use of the obtained polymer in adjusting the pH of a composition containing a polymer obtained by polymerizing a water-soluble unsaturated carboxylic acid monomer in the presence of a water-soluble sucrose allyl ether may decrease the viscosity of the composition at a low pH. The amount of the pentaerythritol allyl ether for use is preferably about 0.1 to 2 parts by mass. The upper limit and the lower limit of the range of the amount may be, for example, 0.15, 0.2, 0.25, 0.3, 0.35, 0.4, 0.45, 0.5, 0.55, 0.6, 0.65, 0.7, 0.75, 0.8, 0.85, 0.9, 0.951, 1.1, 1.2, 1.3, 1.4, 1.5, 1.6, 1.7, 1.8, or 1.9 parts by mass. For example, the amount of the pentaerythritol allyl ether for use may be 0.1 to 1 parts by mass or 0.2 to 0.9 parts by mass. In the polymerization, the pentaerythritol allyl ether can act as a crosslinking agent.

In the polymerization, for example, (meth)acrylic acid alkyl ester wherein the alkyl has 10 to 30 carbon atoms can be preferably used. In other words, a polymer obtained by polymerizing (meth)acrylic acid and (meth)acrylic acid alkyl ester wherein the alkyl has 10 to 30 carbon atoms in the presence of pentaerythritol allyl ether can be preferably used as polymer (B). The amount of (meth)acrylic acid alkyl ester wherein the alkyl has 10 to 30 carbon atoms can be, for example, about 0.5 to 5 parts by mass per 100 parts by mass of (meth)acrylic acid.

The amount of the (meth)acrylic acid alkyl ester for use is 0.5 to 10 parts by mass, preferably 0.5 to 5 parts by mass or 1 to 5 parts by mass, and more preferably 1 to 3 parts by mass, per 100 parts by mass of (meth)acrylic acid.

The (meth)acrylic acid alkyl ester wherein the alkyl has 10 to 30 carbon atoms (10, 11, 12, 13, 14, 15, 16, 17, 18, 19, 20, 21, 22, 23, 24, 25, 26, 27, 28, 29, or 30) refers to an ester of (meth)acrylic acid and a higher alcohol wherein the alkyl group has 10 and 30 carbon atoms. The number of carbon atoms of the alkyl group is more preferably 12 to 30, 14 to 28, 16 to 26, or 18 to 24, for example. Examples of such (meth)acrylic acid alkyl esters include, but are not particularly limited to, esters of (meth)acrylic acid and stearyl alcohol, esters of (meth)acrylic acid and eicosanol, esters of (meth)acrylic acid and behenyl alcohol, and esters of (meth)acrylic acid and tetracosanol. Of these (meth)acrylic acid alkyl esters, stearyl methacrylate, eicosanyl methacrylate, behenyl methacrylate, and tetracosanyl methacrylate are preferable. These (meth)acrylic acid alkyl esters may be used singly, or in a combination of two or more. Commercial products, such as Blemmer VMA70 and Blemmer SMA (trade name, produced by NOF Corporation), may also be used as such (meth)acrylic acid alkyl esters.

The polymerization method for preparing polymer (B) includes, for example, a method of performing polymerization in a polymerization solvent in the presence of a radical polymerization initiator.

Examples of radical polymerization initiators include, but are not limited to, α,α'-azobisisobutyronitrile, 2,2'-azobis-2,4-dimethylvaleronitrile, 2,2'-azobismethylisobutyrate, 2,2'-azobis(2-methylpropionamidine) dihydrochloride, benzoyl peroxide, lauroyl peroxide, cumene hydroperoxide, and tertiary butyl hydroperoxide. These radical polymerization initiators can be used singly, or in a combination of two or more.

The amount of the radical polymerization initiator for use is not particularly limited, and can be, for example, preferably 0.01 to 0.45 parts by mass, and more preferably 0.01 to 0.35 parts by mass, per 100 parts by mass of (meth)acrylic acid.

Although not particularly limited, polymer (B) for use is, for example, preferably the following polymers (B-1) and (B-2).
(B-1) A copolymer obtained by polymerizing the following (i) and (ii) .
(B-2) A copolymer obtained by polymerizing the following (i), (ii), and (iii).
   (i) 100 parts by mass of (meth)acrylic acid.
   (ii) 0.1 to 1 parts by mass of pentaerythritol allyl ether.
   (iii) 0.5 to 5 parts by mass of (meth)acrylic acid alkyl ester wherein the alkyl has 10 to 30 carbon atoms.

Polymer (B) can more preferably be prepared by precipitation polymerization or reversed-phase suspension polymerization. Additionally, reversed-phase suspension polymerization may be performed in a polymerization solvent containing a nonionic surfactant with a polyoxyethylene chain.

The nonionic surfactant with a polyoxyethylene chain is, for example, preferably an ethylene oxide adduct of a polyhydric alcohol fatty acid ester, a block copolymer of hydroxy fatty acid and ethylene oxide, or polyoxyethylene castor oil.

The ethylene oxide adduct of a polyhydric alcohol fatty acid ester is, for example, preferably an ester compound of polyoxyethylene hydrogenated castor oil and polyhydric alcohol fatty acid. The polyhydric alcohol fatty acid is preferably a saturated or unsaturated polyhydric (particularly dihydric) alcohol fatty acid with 14 to 24 carbon atoms (14, 15, 16, 17, 18, 19, 20, 21, 22, 23, or 24). More specifically, the polyhydric alcohol fatty acid is, for example, preferably isopalmitic acid, isostearic acid, or isooleic acid. The average number of moles of added ethylene oxides of polyoxyethylene in the ester compound of polyoxyethylene hydrogenated castor oil and polyhydric alcohol fatty acid is not particularly limited, and can be, for example, about 20 to 100 or about 30 to 70. The ester compound of polyoxyethylene hydrogenated castor oil and polyhydric alcohol fatty acid is particularly preferably polyoxyethylene hydrogenated castor oil isostearate.

The polyoxyethylene castor oil is those with the number of moles of ethylene oxides added being preferably about 2 to 10, and more preferably about 2 to 5.

The block copolymer of hydroxy fatty acid and ethylene oxide can also be rephrased as a copolymer of polyhydroxy fatty acid and polyoxyethylene. The fatty acid of polyhydroxy fatty acid is preferably a fatty acid with about 14 to 22 carbon atoms, preferably myristic acid, palmitic acid, or stearic acid. The hydroxy fatty acid is preferably hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably polyhydroxystearic acid. The block copolymer of hydroxy fatty acid and ethylene oxide is particularly preferably a block copolymer of 12-hydroxystearic acid and ethylene oxide.

The nonionic surfactant with a polyoxyethylene chain for use can be a single nonionic surfactant, or a combination of two or more nonionic surfactants.

The amount of the nonionic surfactant with a polyoxyethylene chain for use is preferably about 0.5 to 10 parts by mass per 100 parts by mass of (meth)acrylic acid (i). The lower limit of the amount in parts by mass may be, for example, about 0.75, 1.0, 1.25, or 1.5, and the upper limit of the amount in parts by mass may be, for example, about 9.5, 9, 8.5, 8, 7.5, 7, 6.5, 6, or 5.5. For example, the amount of the nonionic surfactant with a polyoxyethylene chain for use is more preferably about 1 to 7.5 parts by mass.

Although not particularly limited, the polymerization solvent is preferably a solvent in which (meth)acrylic acid, the (meth)acrylic acid alkyl ester, or pentaerythritol allyl ether is dissolved, but obtained polymer (B) is not dissolved. Specific examples of such polymerization solvents include n-pentane, n-hexane, n-heptane, n-octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Of these polymerization solvents, from the standpoint of stable quality and availability, ethylene dichloride, n-hexane, n-heptane, and ethyl acetate are preferable. These polymerization solvents can be used singly, or in a combination of two or more.

The amount of the polymerization solvent for use is, for example, but is not limited to, preferably 200 to 10000 parts by mass, and more preferably 300 to 2000 parts by mass, per 100 parts by mass of (meth)acrylic acid.

The atmosphere for performing the polymerization reaction is not particularly limited as long as the polymerization reaction can be performed; the atmosphere is, for example, an inert gas atmosphere such as nitrogen gas or argon gas.

The reaction temperature for the polymerization reaction is not particularly limited as long as the polymerization reaction can be performed; the reaction temperature is, for example, preferably 50 to 90°C, and more preferably 55 to 75°C. Performing a polymerization reaction at these reaction temperatures suitably limits the increase in viscosity of the reaction solution, simplifies reaction control, and further allows for control of the bulk density of the resulting polymer (B).

The reaction time for the polymerization reaction cannot be generalized because it varies according to the reaction temperature; however, the reaction time is typically 2 to 10 hours.

After completion of the reaction, polymer (B) in a fine white powder form can be isolated, for example, by heating the reaction solution to 80 to 130°C to remove the polymerization solvent.

The degree of neutralization of polymer (B) can also be easily adjusted by neutralizing the carboxyl groups of acrylic acid and/or methacrylic acid with an alkali. The phrase "degree of neutralization" as used here refers to the percentage of the number of moles of neutralized groups of the total number of moles of carboxyl groups of acrylic acid and/or methacrylic acid. Examples of alkalis for use in neutralization include sodium hydroxide, potassium hydroxide, triethanolamine, and diisopropylamine. The neutralization method is not particularly limited and is, for example, a method of neutralizing acrylic acid and/or methacrylic acid beforehand, or a method of neutralizing the polymer obtained by polymerization.

Although not particularly limited, the degree of neutralization of polymer (B) is preferably lower than the degree of neutralization of polymer (A). In other words, the degree of neutralization of polymer (A) is preferably higher than the degree of neutralization of polymer (B). This is because it is preferable to use polymer (B) with a degree of neutralization lower than that of polymer (A) in adjusting the pH of the composition containing polymer (A) with polymer (B), especially when the pH of the composition containing polymer (A) is to be reduced. The degree of neutralization of polymer (B) is, for example, preferably 70% or lower or 60% or lower, more preferably 50% or lower or 45% or lower, and still more preferably 40% or lower. Polymer (B) that is not neutralized (degree of neutralization: 0%) is also preferable.

The water contained in the composition of the present disclosure is not particularly limited. For example, the water for use can be tap water, distilled water, or ion-exchanged water.

The pH of the composition of the present disclosure is 5.2 or lower, and for example, preferably about 3.5 to 5.2. The upper limit and the lower limit of these ranges may be, for example, 3.6, 3.7, 3.8, 3.9, 4, 4.1, 4.2, 4.3, 4.4, 4.5, 4.6, 4.7, 4.8, 4.9, 5, or 5.1. For example, the pH may be about 4 to 5.1. The pH is measured with a pH meter.

The viscosity of the composition of the present disclosure is 1000 mPa·s or higher, and is, for example, preferably about 1000 to 50000 mPa·s. The upper limit and the lower limit of these ranges may be, for example, 1500, 2000, 2500, 3000, 3500, 4000, 4500, 5000, 5500, 6000, 6500, 7000, 7500, 8000, 8500, 9000, 9500, 10000, 11000, 12000, 13000, 14000, 15000, 16000, 17000, 18000, 19000, 20000, 21000, 22000, 23000, 24000, 25000, 26000, 27000, 28000, 29000, 30000, 31000, 32000, 33000, 34000, 35000, 36000, 37000, 38000, 39000, 40000, 41000, 42000, 43000, 44000, 45000, 46000, 47000, 48000, or 49000 mPa·s. For example, the range of viscosity may be 1500 to 39000 mPa·s. The viscosity is measured with a BH rotatory viscometer. Specifically, the viscosity is measured by reading the viscosity value one minute after the rotor starts rotating at 25°C with a rotation speed of the spindle rotor at 20 rpm. As a guide, the rotor used in measurement is as follows: rotor No.3 for lower than 2000 mPa·s, rotor No.4 for 2000 mPa·s or higher and lower than 5000 mPa·s, rotor No.5 for 5000 mPa·s or higher and lower than 15000 mPa·s, rotor No.6 for 15000 mPa·s or higher and lower than 40000 mPa·s, and rotor No.7 for 40000 mPa.s or higher. Optionally, each viscous composition can be defoamed by centrifugation (e.g., 2000 rpm for 10 to 20 minutes) before viscosity measurement.

The composition of the present disclosure is useful, for example, as a cosmetic composition.

The composition of the present disclosure may contain components other than those listed above to the extent that the effects of the composition are not impaired; for example, the composition may contain components known in the field of cosmetics. For example, the composition may contain a thickening agent to the extent that the effects of the composition are not impaired. Examples of thickening agents include polysaccharide-based water-soluble polymers (e.g., hydroxyethyl cellulose and xanthan gum), carboxyvinyl polymers, and alkyl-modified carboxyvinyl polymers. The composition of the present disclosure may also contain, for example, powder components, liquid fats and oils, solid fats and oils, wax, hydrocarbon oils, higher fatty acids, higher alcohols, ester oils, silicone oils, anionic surfactants, cationic surfactants, ampholytic surfactants, nonionic surfactants, moisturizing agents, water-soluble polymers, film agents, UV absorbers, sequestrants, lower alcohols, polyhydric alcohols, carbohydrates, amino acids, organic amines, polymer emulsions, pH adjusters, skin nutrients, vitamins, antioxidants, antioxidant aids, and fragrances.

The composition of the present disclosure for use in the field of cosmetics is not particularly limited in terms of the form. Examples of the form of the composition for use in the field of cosmetics include medicated creams, lotions, milky lotions, serums, creams, cream packs, massage creams, cleansing creams, cleansing gels, gel creams, facial cleansing foams, moisturizing gels, hair setting gels, sunscreens, styling gels, eyeliners, mascaras, lipsticks, and foundations

In the present specification, the term "comprising" includes the concepts of consisting essentially of and consisting of. The present disclosure also encompasses any and every combination of the elements described in the present specification.

The various properties (characteristics, structures, functions, etc.) described above for each embodiment of the present disclosure may be combined in any way in identifying the subject matter encompassed by the present disclosure. Specifically, the disclosure encompasses all subject matter formed of any and every combination of the combinable properties described in the specification.

### Examples

The following describes embodiments of the present disclosure in more detail with reference to Examples. However, the embodiments of the disclosure are not limited to the Examples below.

### Preparation of Polymer (A)

### Synthesis of Water-soluble Sucrose Allyl Ether

### Production Example 1

A stirrer, a reflux condenser, and a dropping funnel were attached to a 1000-mL separable flask. In the separable flask, 48 g (1.2 mol) of sodium hydroxide was dissolved in 144 g of water. 136.8 g (0.4 mol) of sucrose was added thereto, and the mixture was stirred at 70 to 85°C for 120 minutes, thereby preparing an aqueous solution of alkaline sucrose. 145.2 g (1.2 mol) of allyl bromide was added dropwise to the aqueous solution of alkaline sucrose at 70 to 85°C over a period of 1.5 hours, and the mixture was then aged at 80°C for 3 hours to allyl-etherify the sucrose. After cooling, 440 g of water was added, and excess oil was separated through a separatory funnel, thereby obtaining an aqueous solution of crude sucrose allyl ether. Hydrochloric acid was added to the aqueous solution of crude sucrose allyl ether to adjust the pH to 6 to 8, and then water was removed by using a rotary evaporator until the mass of the aqueous solution reached 480 g. Subsequently, 200 g of ethanol was added to precipitate salts such as sodium bromide (by-products), and the precipitates were removed from the aqueous solution by filtration. The excess water was further removed from the aqueous solution by using an evaporator, thereby obtaining 166 g of a purified water-soluble sucrose allyl ether with a degree of etherification of 2.4.

### Synthesis of Resin (a polymer of a water-soluble unsaturated carboxylic acid monomer crosslinked by water-soluble sucrose allyl ether)

### Production Example 2

A stirrer, a reflux condenser, and a dropping funnel were attached to a 500-mL separable flask. 72 g of acrylic acid and water were added to the separable flask, thereby preparing 90 g of an 80 mass% aqueous solution of acrylic acid. Further, 56 g of ion-exchanged water, 0.023 g of the water-soluble sucrose allyl ether of Production Example 1 as a crosslinking agent (0.25 mass% based on the aqueous solution of acrylic acid), and 0.04 g of 2,2'-azobis(2-methylpropionamidine) dihydrochloride (V-50, produced by Wako Pure Chemical Industries, Ltd.) as an initiator were added, thereby preparing an aqueous solution of a water-soluble unsaturated carboxylic acid monomer.

Separately, 330 g of n-heptane was placed in a 2-L separable flask equipped with a stirrer, a reflux condenser, a dropping funnel, and a nitrogen gas-feeding tube. Further, 1.0 g of a maleic anhydride-modified ethylene-propylene copolymer (Hi-WAX 1105A, Mitsui Chemicals, Inc.) and 1.0 g of sucrose stearate with an HLB of 3 (RYOTO sugar ester S-370, Mitsubishi-Kagaku Foods Corporation) as a surfactant were added to the flask and dispersed and dissolved in n-heptane. The aqueous solution of a water-soluble unsaturated carboxylic acid monomer prepared beforehand was then added thereto. While nitrogen gas was blown into the solution for nitrogen purging of the system in order to eliminate the atmosphere inside the reactor and oxygen present in the starting materials and in the solvent, polymerization was performed according to reversed-phase suspension polymerization over a period of 1 hour at a bath temperature maintained at 60°C with stirring at a stirring rate of 1000 rpm. After completion of polymerization, the water and n-heptane were distilled off, thereby obtaining 67 g of a resin powder, which is a polymer of acrylic acid and a sodium salt thereof, crosslinked by water-soluble sucrose allyl ether, with a degree of neutralization of 0% (this resin may be referred to as "polymer 2" below).

### Production Example 3

A stirrer, a reflux condenser, and a dropping funnel were attached to a 500-mL separable flask. 72 g of acrylic acid and water were add to the separable flask, thereby preparing 90 g of an 80 mass% aqueous solution of acrylic acid. While the aqueous solution of acrylic acid was cooled, 27 g of a 30 mass% aqueous solution of sodium hydroxide was added dropwise to neutralize the aqueous solution. Further, 56 g of ion-exchanged water, 0.023 g of the water-soluble sucrose allyl ether of Production Example 1 as a crosslinking agent (0.25 mass% based on the aqueous solution of acrylic acid), and 0.04 g of 2,2'-azobis(2-methylpropionamidine) dihydrochloride (V-50, produced by Wako Pure Chemical Industries, Ltd.) as an initiator were added, thereby preparing an aqueous solution of a water-soluble unsaturated carboxylic acid monomer.

Separately, 330 g of n-heptane was placed in a 2-L separable flask equipped with a stirrer, a reflux condenser, a dropping funnel, and a nitrogen gas-feeding tube. Further, 1.0 g of a maleic anhydride-modified ethylene-propylene copolymer (Hi-WAX 1105A, Mitsui Chemicals, Inc.) as a surfactant, and 1.0 g of sucrose stearate with an HLB of 3 (RYOTO sugar ester S-370, Mitsubishi-Kagaku Foods Corporation) as a surfactant were added to the flask and dispersed and dissolved in n-heptane. The aqueous solution of a water-soluble ethylene unsaturated monomer prepared beforehand was then added thereto. While nitrogen gas was blown into the solution for nitrogen purging of the system in order to eliminate the atmosphere inside the reactor and oxygen present in the starting materials and in the solvent, polymerization was performed according to reversed-phase suspension polymerization over a period of 1 hour at a bath temperature maintained at 60°C with stirring at a stirring rate of 1000 rpm. After completion of polymerization, the water and n-heptane were distilled off, thereby obtaining 71 g of a resin powder, which is a polymer of acrylic acid and a sodium salt thereof, crosslinked by water-soluble sucrose allyl ether, with a degree of neutralization of 20% (this resin may be referred to as "polymer 3" below).

### Production Example 4

The procedure of Production Example 3 was repeated, except that the amount of the 30 mass% aqueous solution of sodium hydroxide was changed to 54 g, thereby obtaining 75 g of a resin powder with a degree of neutralization of 40% (this resin may be referred to as "polymer 4").

### Production Example 5

The procedure of Production Example 4 was repeated, except that the stirring rate was changed to 800 rpm, thereby obtaining 75 g of a resin powder with a degree of neutralization of 40% (this resin may be referred to as "polymer 5").

### Production Example 6

The procedure of Production Example 4 was repeated, except that the stirring rate was changed to 600 rpm, thereby obtaining 75 g of a resin powder with a degree of neutralization of 40% (this resin may be referred to as "polymer 6").

### Production Example 7

The procedure of Production Example 3 was repeated, except that the amount of the 30 mass% aqueous solution of sodium hydroxide was changed to 94 g, thereby obtaining 81 g of a resin powder with a degree of neutralization of 70% (this resin may be referred to as "polymer 7").

### Evaluation of Resin Properties

### Measurement of Median Particle Size

The resins obtained in Production Examples 2 to 7 (polymers 2 to 7) were individually dispersed in n-hexane and measured for the median particle size of volume average particle size with a laser particle size analyzer (produced by Shimadzu Corporation) (using a SALD2000 flow cell). Table 1 shows the results.

### Measurement of Viscosity of 0.5 Mass% Aqueous Solution

The resins (polymers 2 to 7) were individually mixed with water to prepare aqueous solutions (concentration: 0.5 mass%). For a resin containing an acrylic acid sodium salt as a monomer unit, the percent by mass of the resin was calculated based on the mass of the monomer unit of acrylic acid. The pH of the aqueous solutions containing polymers 3 to 7 was 4.8 to 7.0. The aqueous solutions containing resin A and a carboxyvinyl polymer were adjusted to have a pH of 6.0 with a predetermined amount of a 6 mass% aqueous solution of sodium hydroxide (neutralizer), and to have a resin concentration of 0.5 mass%. The viscosity of each 0.5 mass% aqueous solution was measured with a BH rotatory viscometer. The viscosity value was read one minute after the rotor started rotating at 25°C with a rotation speed of the spindle rotor at 20 rpm. For resins 2 to 7, a rotor No. 6 was used. Table 1 shows the results.

**Table 1**

| | | Degree of Neutralization (%) | Median Particle Size (µm) | Viscosity of 0.5 Mass% Aqueous Solution (mPa·s) | pH |
|---|---|---|---|---|---|
| Production Example 2 | Polymer 2 | 0 | 7 | 18,000 | 6.0 |
| Production Example 3 | Polymer 3 | 20 | 8 | 16,000 | 4.8 |
| Production Example 4 | Polymer 4 | 40 | 10 | 15,000 | 6.0 |
| Production Example 5 | Polymer 5 | 40 | 20 | 13,000 | 6.0 |
| Production Example 6 | Polymer 6 | 40 | 35 | 12,000 | 6.0 |
| Production Example 7 | Polymer 7 | 70 | 10 | 14,000 | 7.0 |

### Preparation of Polymer (B)

### Production Example a

45 g (0.625 mol) of acrylic acid, 0.27 g of pentaerythritol tetraallyl ether (PETA), 150 g of n-hexane, and 0.081 g (0.00035 mol) of 2,2'-azobismethyl isobutyrate were placed in a 500-mL four-necked flask equipped with a stirrer, thermometer, a nitrogen gas-feeding tube, and a condenser to prepare a reaction solution. The solution was homogeneously mixed with stirring, and then nitrogen gas was blown into the solution to eliminate the oxygen present in the upper space of the reactor, in the starting materials, and in the solvent. Subsequently, the reaction solution was maintained at 60 to 65°C to allow a reaction to proceed for 4 hours in a nitrogen atmosphere. After completion of the reaction, the generated slurry was heated to 90°C to distill off n-hexane and further dried under reduced pressure at 110°C at 10 mmHg for 8 hours, thereby obtaining 42 g of a carboxyvinyl polymer. This carboxyvinyl polymer may be referred to as "polymer a" below.

### Production Example b

40 g of acrylic acid, 0.22 g of pentaerythritol tetraallyl ether, 0.116 g of 2,2'-azobis(methylisobutyrate), and 230.9 g of n-hexane were placed in a 500-mL (milliliter) four-necked flask equipped with a stirrer, thermometer, a nitrogen gas-feeding tube, and a condenser. The solution was homogeneously mixed with stirring, and then nitrogen gas was blown into the solution to eliminate the oxygen present in the upper space of the reactor (four-necked flask), in the starting materials, and in the solvent. Subsequently, the solution was heated to 60 to 65°C in a nitrogen atmosphere. The solution was then maintained at 60 to 65°C for 3 hours. At the point that the solution had been maintained at 60 to 65°C for about 1 hour, a solution of 2.0 g of a block copolymer of 12-hydroxystearic acid and polyoxyethylene (Hypermer B246, produced by Croda) in 2.0 g of n-hexane was added to the reactor. Thereafter, the generated slurry was heated to 100°C to distill off n-hexane and further dried under reduced pressure at 115°C at 10 mmHg for 8 hours, thereby obtaining 38 g of a carboxyvinyl polymer in a fine white powder form. This carboxyvinyl polymer may be referred to as "polymer b" below.

### Production Example c

40 g of acrylic acid, 0.4 g of Blemmer VMA-70 (produced by NOF Corporation; a mixture of 10 to 20 parts by mass of stearyl methacrylate, 10 to 20 parts by mass of eicosanyl methacrylate, 59 to 80 parts by mass of behenyl methacrylate, and tetracosanyl methacrylate in an amount of 1 mass% or less), 0.24 g of pentaerythritol tetraallyl ether, 0.116 g of 2,2'-azobis(methylisobutyrate), and 230.9 g of n-hexane were placed in a 500-mL (milliliter) four-necked flask equipped with a stirrer, thermometer, a nitrogen gas-feeding tube, and a condenser. The solution was homogeneously mixed with stirring, and then nitrogen gas was blown into the solution to eliminate the oxygen present in the upper space of the reactor (four-necked flask), in the starting materials, and in the solvent. Subsequently, the solution was heated to 60 to 65°C in a nitrogen atmosphere. The solution was then maintained at 60 to 65°C for 3 hours. At the point that the solution had been maintained at 60 to 65°C for about 1 hour, a solution of 1.6 g of a block copolymer of 12-hydroxystearic acid and polyoxyethylene (Hypermer B246, produced by Croda) in 2.0 g of n-hexane was added to the reactor. Thereafter, the generated slurry was heated to 100°C to distill off n-hexane and further dried under reduced pressure at 115°C at 10 mmHg for 8 hours, thereby obtaining 39 g of a water-soluble copolymer containing alkyl-modified carboxyl groups in a fine white powder form. This copolymer may be referred to as "polymer c" below.

The uncrosslinked acrylic polymer for use was purchased. Specifically, Polyacrylic Acid 5,000 and Polyacrylic Acid 25,000 (produced by Wako Pure Chemical Industries, Ltd.) were purchased. The former uncrosslinked polyacrylic acid had a weight average molecular weight of 5000, and the latter uncrosslinked polyacrylic acid had a weight average molecular weight 25000. The former may be referred to as "polymer d," and the latter may be referred to as "polymer e" below.

### Production Example f

45 g (0.625 mol) of acrylic acid, 1.35 g of Blemmer VMA70 (a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate; produced by NOF Corporation), 0.02 g of pentaerythritol tetraallyl ether, 150 g of n-hexane, and 0.081 g (0.00035 mol) of 2,2'-azobismethyl isobutyrate were placed in a 500-mL four-necked flask equipped with a stirrer, a thermometer, a nitrogen gas-feeding tube, and a condenser. The solution was homogeneously mixed with stirring, and then nitrogen gas was blown into the solution to eliminate the oxygen present in the upper space of the reactor (four-necked flask), in the starting materials, and in the solvent. Subsequently, the solution was then allowed to react for 4 hours in a nitrogen atmosphere while being maintained at 60 to 65°C. After completion of the reaction, the generated slurry was heated to 90°C to distill off n-hexane and further dried under reduced pressure at 110°C at 10 mmHg for 8 hours, thereby obtaining 43 g of a water-soluble copolymer containing alkyl-modified carboxyl groups in a fine white powder form. The copolymer may be referred to as "polymer f" below.

### Preparation and Evaluation of Viscose Composition

Polymers a to f were individually dissolved or dispersed in ion-exchanged water to give a concentration of 0.5 mass%, thereby obtaining aqueous polymer solutions. Separately, polymer 4 was mixed with ion-exchanged water in a concentration of 0.5 mass% to prepare a gel. The 0.5 mass% aqueous solutions of the polymers (polymers a to f) were individually added to 100 g of the gel in increments of 10 g, thereby preparing viscous compositions of a different pH. Additionally, 1 g, 2 g, or 5 g of an aqueous solution of citric acid (1 mass%) was added to 100 g of the gel, thereby preparing viscous compositions of a different pH.

The obtained viscous compositions were measured for viscosity and pH. The viscosity was measured with a BH rotatory viscometer. A viscosity value was read one minute after the rotor started rotating at 25°C with a rotation speed of the spindle rotor at 20 rpm. A rotor No.5, No.6, or No.7 was used. The viscous compositions were defoamed by centrifugation (2000 rpm for 10 to 20 minutes) before viscosity measurement. The pH was measured with a pH meter. Fig. 1 shows the results.

Table 2 summarizes some of the results.

**Table 2**

| | Polymer (B) | Polymer (A) | Viscosity (pH≤5.4) | Formulation of Polymer (B) (Molar Ratio) | | |
|---|---|---|---|---|---|---|
| | | | A: 10,000≤ B: 10,000> | Acrylic Acid | Blemmer VMA70 | PETA |
| Example 1 | Polymer a | Polymer 4 | A | 100 | - | 0.6 |
| Example 2 | Polymer b | Polymer 4 | A | 100 | - | 0.55 |
| Example 3 | Polymer c | Polymer 4 | A | 100 | 1 | 0.6 |
| Comparative Example 1 | Polymer d | Polymer 4 | B | 100 | - | - |
| Comparative Example 2 | Polymer e | Polymer 4 | B | 100 | - | - |
| Comparative Example 3 | Polymer f | Polymer 4 | B | 100 | 3 | 0.044 |
| Reference Example 1 | Citric Acid (Low-molecular Organic Acid Compound) | Polymer 4 | B | - | - | - |

From the results above, the following was found. When adjusting (in particular, reducing) the pH of a polymer obtained by polymerizing a water-soluble unsaturated carboxylic acid monomer in the presence of a water-soluble sucrose allyl ether, the use of a low-molecular organic acid compound decreases the viscosity. However, the use of a polymer obtained by polymerizing at least acrylic acid and/or methacrylic acid in the presence of pentaerythritol allyl ether does not decrease the viscosity. Additionally, the effect was also found to be difficult to achieve even with such a polymer obtained by polymerizing at least acrylic acid and/or methacrylic acid in the presence of pentaerythritol allyl ether unless the amount of pentaerythritol allyl ether is about 0.1 parts by mass or more per 100 parts by mass of the acrylic acid and/or methacrylic acid.

Examples of formulations are shown below.

**Table 3**

| | VC Serum Gel for Acne | | |
|---|---|---|---|
| | Displayed Name | Trade Name | Amount (Parts by Mass) |
| A Room Temperature | Ethoxydiglycol | Transcutol CG | 4 |
| | Methylparaben | Methylparaben | 0.1 |
| | Bis-ethoxydiglycol Cyclohexane-1,4-Dicarboxylate | Neosolue-Aqulio | 1 |
| | BG | BG | 2 |
| | Glycerin | Glycerin | 8 |
| | Sodium Carbomer | Polymer 4 | 0.2 |
| | Acrylate/Alkyl Acrylate (C10-30) Crosspolymer | Polymer c | 0.8 |
| B Room Temperature | 10% Aqueous Solution of Citric Acid | Citric Acid | 0.85 |
| C Room Temperature | Pentetic Acid 0.05*393/504=0.04 | Chelest PA-SD | 0.04 |
| | Tetrahydroxypropyl Ethylenediamine | ADEKA CARPOL MD-100 | 0.35 |
| | Water | Water | 5 |
| D 80°C | Water | Water | 62.88 |
| E 35°C | Ascorbyl Ethyl, Water | VC Ethyl (10% Aqueous Solution) | 10 |
| F 35°C | Water, Papain, Carbomer, Sodium Alginate 1,2-hexanediol, Caprylyl Glycol | X-Pressin (produced by BASF) | 1 |
| | Phenoxyethanol | Phenoxyethanol | 0.2 |
| G 35°C | BG | BG | 2 |
| | PPG-6-Decyltetradeceth-30 | NIKKOL PEN-4630 | 0.3 |
| | PEG-60 Hydrogenated Castor Oil | NIKKOL HCO-60 | 0.3 |
| | 10-Hydroxydecanoic Acid, Sebacic Acid, 1,10-Decanediol, BG | AcnAcidol BG | 1 |
| | Bergamot Oil | Bergamot Oil Bergapten Free (produced by Yamamoto Perfumery Co., Ltd.) | 0.02 |
| | Lemon Oil | Lemon Oil Furocoumarin Free (produced by Yamamoto Perfumery Co., Ltd.) | 0.01 |
| | | Total | 100 |
| | | pH | 4.46 |
| | | Viscosity, mPA·s | 1800 |
| | | Transparency | Translucent |

| | | | |
|---|---|---|---|
| 1) A is dispersed at room temperature and allowed to stand for 10 minutes to allow a thickening agent to swell. 2) B is added to A at room temperature. 3) C is dissolved at room temperature beforehand and added to (A+B) at room temperature. 4) D is added to (A+B+C) at room temperature and stirred at 80°C for 30 minutes to dissolve a thickening agent to increase viscosity. 5) The mixture is cooled to 35°C, and evaporative water is replenished. 6) E is dissolved at room temperature beforehand and added to (A+B+C+D) at room temperature. 7) Each component of F is added to (A+B+C+D+E) sequentially. 8) A solubilizer solution is dissolved by heating beforehand, and oil components such as fragrances (bergamot oil, lemon oil) are dissolved in the solubilizer solution to form a G phase. G is added to (A+B+C+D+E+F) to solubilize it. | | | |

**Table 4**

| | Mild Exfoliating Cleansing Gel | | |
|---|---|---|---|
| | Displayed Name | Trade Name | Amount (Parts by Mass) |
| A 70°C | Coconut Fatty Acid PEG-7 Glyceryl | Hyber Oil HE | 20 |
| | PEG-10 Hydrogenated Castor Oil | EMALEX HC-10 | 5 |
| | DPG | DPG | 5 |
| | Diglycerin | Diglycerin | 1 |
| | Sodium Carbomer | Polymer 4 | 0.2 |
| B 70°C | Water | Water | 46.03 |
| | Pullulan | Pullulan for Cosmetics (Hayashibara Co., Ltd.), Polysaccharide | 0.01 |
| | Fructose | Pure Fructose (Kato Kagaku Co., Ltd.; Kankosha Co., Ltd.) | 0.2 |
| | Glucose | Glu Final 2000 (Kankosha Co., Ltd.) | 0.2 |
| | Betaine | Aminocoat (Asahi Kasei Finechem Co., Ltd.) | 0.2 |
| | 10% Aqueous Solution of Citric Acid | Citric Acid | 0.5 |
| | BG | BG | 5 |
| C 70°C | (Acrylate/Alkyl Acrylate (C10-30)) Crosspolymer | 5% Aqueous Dispersion of Polymer c | 16 |
| D 35°C | Phenoxyethanol | Caflect PE-1 | 0.18 |
| | Silver Oxide, Pentasodium Pentetate, Phytic Acid | Jewel Silver | 0.25 |
| | Sodium Hyaluronate | Bio-sodium Hyaluronate HA20N 1% (Shiseido Company, Ltd.) | 0.05 |
| | Water-soluble Collagen | Porcine Atelocollagen 1% PE (Koken Co., Ltd.) | 0.1 |
| | Tocopherol | Tocopherol | 0.05 |
| | Bergamot Oil | Bergamot Oil Bergapten Free (produced by Yamamoto Perfumery Co., Ltd.) | 0.02 |
| | Lemon Oil | Lemon Oil Furocoumarin Free (produced by Yamamoto Perfumery Co., Ltd.) | 0.01 |
| | | Total | 100.00 |
| | | pH | 4.50 |
| | | Viscosity, mPa·s | 11000 |

| | | | |
|---|---|---|---|
| 1) A is mixed and heated to 70°C. 2) B is mixed and heated to 70°C. 3) B is added to A at 70°C. 4) C is added to (A+B) at 70°C. 5) The mixture is cooled to 35°C or below. 6) Each component of D is added to (A+B+C) sequentially. | | | |

**Table 5**

| | Deodorant Cream, Solid Perfume | | |
|---|---|---|---|
| | Displayed Name | Trade Name | Amount (Parts by Mass) |
| A | Glyceryl Caprylate | Sunsoft No.700P-2-C (Taiyo Kagaku Co., Ltd.) | 1 |
| | Cetearyl Alcohol | Kalcol 6850 (Kao Corporation.) | 4 |
| | Zinc Ricinoleate | TEGODEO PY 88 (Evonik) | 2 |
| | Glyceryl Stearate Citrate | IMWITOR 372 P | 3 |
| | Isoamyl Laurate | Dermofeel Sensolv (Evonik) | 6 |
| | Triethyl Citrate | CITROFLEX-2 (Vertellus Specialties) | 2 |
| | Sodium Carbomer | Polymer 4 | 0.3 |
| B | Water | Water | 14.62 |
| | Methylparaben | Methylparaben | 0.2 |
| | (Acrylate/Alkyl Acrylate (C10-30)) Crosspolymer | 2% Aqueous Dispersion of Polymer c | 60 |
| C | Glycerin | Concentrated Glycerin (Miyoshi Oil & Fat Co., Ltd.) | 3 |
| | Talc | FG106 (Fuji Talc Industrial Co., Ltd. ) | 2 |
| | Water | Water | 1 |
| D | Malic Acid, Water | 10% Malic Acid | 0.5 |
| E | Phenoxyethanol | Phenoxyethanol | 0.18 |
| | Fragrance | S.C.197-4792 Floral Soap | 0.2 |
| | | Total | 100 |
| | | pH | 4.37 |
| | | Viscosity, mPa·s | 761000 |
| | | Form | Paste |

| | | | |
|---|---|---|---|
| 1) A is mixed at 80°C. 2) B is mixed at 80°C. 3) B is added to A at 80°C and stirred with a homomixer for 1 to 2 minutes. 4) The mixture is cooled to 40°C. 5) C is added to (A+B). 6) The mixture is cooled to room temperature. 7) D is added to (A+B+C) to adjust the pH to 4.2-4.5. 8) E is added to (A+B+C+D). | | | |

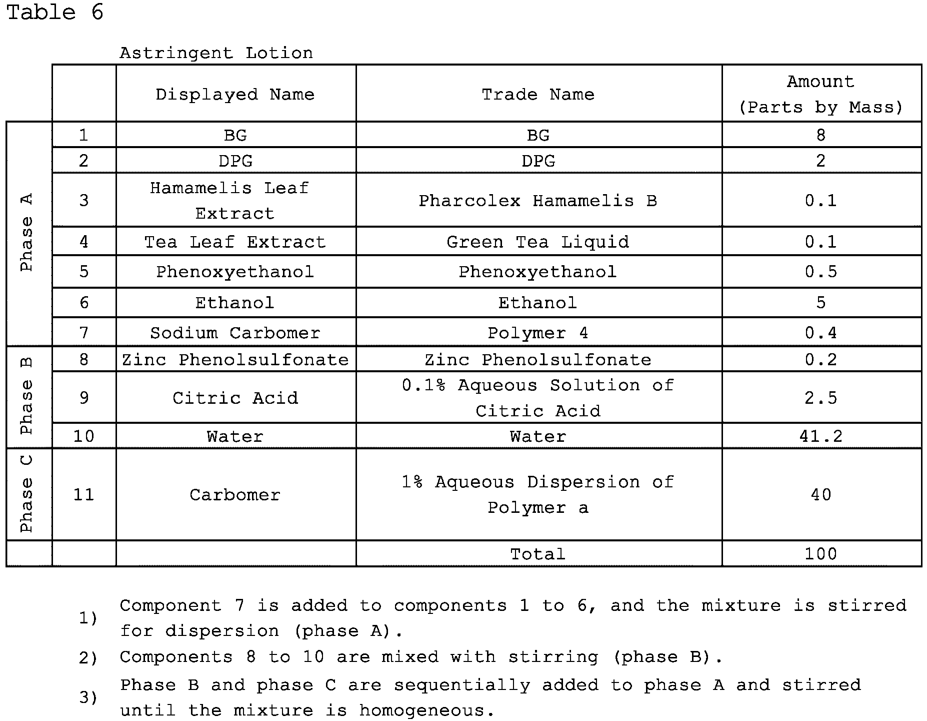

## Claims

1. A composition comprising
(A) a polymer obtained by polymerizing a water-soluble unsaturated carboxylic acid monomer in the presence of a water-soluble sucrose allyl ether,
(B) a polymer obtained by polymerizing at least acrylic acid and/or methacrylic acid in the presence of pentaerythritol allyl ether, wherein the amount of the pentaerythritol allyl ether is 0.1 parts by mass or more per 100 parts by mass of the acrylic acid and/or methacrylic acid, and
water,
the composition having a pH of 5.2 or lower and a viscosity of 1000 mPa·s or higher.

2. The composition according to claim 1, wherein the water-soluble unsaturated carboxylic acid monomer in component (A) is at least one member selected from the group consisting of acrylic acid, methacrylic acid, maleic acid, maleic anhydride, fumaric acid, crotonic acid, and itaconic acid.

3. The composition according to claim 1 or 2, wherein the amount of the pentaerythritol allyl ether in component (B) is 0.1 to 1 parts by mass per 100 parts by mass of the acrylic acid and/or methacrylic acid.

4. The composition according to any one of claims 1 to 3, having a pH of 4 to 5.2.

5. The composition according to any one of claims 1 to 4, having a viscosity of 1000 to 50000 mPa·s.

6. The composition according to any one of claims 1 to 5, wherein the amount of the water-soluble sucrose allyl ether in component (A) is 0.01 to 1 parts by mass per 100 parts by mass of the water-soluble unsaturated carboxylic acid monomer.

7. The composition according to any one of claims 1 to 6, wherein component (B) is the following copolymer (B-1) and/or copolymer (B-2):
(B-1) a copolymer obtained by polymerizing the following (i) and (ii), and
(B-2) a copolymer obtained by polymerizing the following (i), (ii), and (iii);
(i) 100 parts by mass of (meth)acrylic acid,
(ii) 0.1 to 1 parts by mass of pentaerythritol allyl ether, and
(iii) 0.5 to 5 parts by mass of (meth)acrylic acid alkyl ester wherein the alkyl has 10 to 30 carbon atoms.

8. A pH adjuster for a composition,
the pH adjuster comprising (B) a polymer obtained by polymerizing a water-soluble unsaturated carboxylic acid monomer in the presence of pentaerythritol allyl ether, wherein the amount of the pentaerythritol allyl ether is 0.1 parts by mass or more per 100 parts by mass of the water-soluble unsaturated carboxylic acid monomer,
the composition comprising (A) a polymer obtained by polymerizing a water-soluble unsaturated carboxylic acid monomer in the presence of a water-soluble sucrose allyl ether.
